# EUROPÄISCHE PATENTSCHRIFT

(11) **EP 0 800 507 B1**
(45) Veröffentlichungstag und Bekanntmachung des Hinweises auf die Patenterteilung: **23.06.1999**
(21) Anmeldenummer: 95941717.1
(22) Anmeldetag: 16.12.1995
(51) Int. Cl.: C07C 253/30, C07C 255/24

(54) **VERFAHREN ZUR HERSTELLUNG VON ALIPHATISCHEN ALPHA, OMEGA-AMINONITRILEN**
PROCESS FOR PREPARING ALIPHATIC ALPHA,OMEGA-AMINONITRILES
PROCEDE DE PREPARATION D'AMINONITRILES-ALPHA,OMEGA ALIPHATIQUES

(30) Priorität: 27.12.1994 DE 4446894
(43) Veröffentlichungstag der Anmeldung: 15.10.1997
(73) Patentinhaber: BASF AKTIENGESELLSCHAFT, 67056 Ludwigshafen (DE)
(72) Erfinder: SCHNURR, Werner, D-67273 Herxheim (DE); FISCHER, Rolf, D-69121 Heidelberg (DE); BASSLER, Peter, D-68519 Viernheim (DE); HARDER, Wolfgang, D-69469 Weinheim (DE)
(86) Internationale Anmeldenummer: EP9504984
(87) Internationale Veröffentlichungsnummer: WO9620165

(56) Entgegenhaltungen:
- WO-A-92/21650
- WO-A-93/16034
- US-A- 5 151 543

## Beschreibung

Die vorliegende Erfindung betrifft ein verbessertes Verfahren zur Herstellung von aliphatischen alpha,omega-Aminonitrilen durch partielle Hydrierung von aliphatischen alpha,omega-Dinitrilen bei erhöhter Temperatur und erhöhtem Druck in Gegenwart einer Base und eines Hydrierungs-Katalysators.

Die US 5,151,543 beschreibt die partielle Hydrierung von aliphatischen Dinitrilen zu den entsprechenden Aminonitrilen in Gegenwart von Raney-Nickel-Katalysatoren. Als Lösungsmittel wird nach diesem Verfahren entweder Ammoniak oder ein Alkohol verwendet, wobei beim Einsatz von Alkohol zusätzlich eine anorganische Base erforderlich ist. Es wird in der US 5,151,543 ausdrücklich beschrieben, daß bei Verwendung von Ammoniak als Lösungsmittel keine weitere Base erforderlich ist.

Insbesondere ist bei dem Verfahren nach der US 5,151,543 von Nachteil, daß bei Verwendung von Ammoniak die Ausbeute, je nach Umsatz, nur 60 bis 62% beträgt, und verhältnismäßig große Mengen an Hexamethylendiamin gebildet werden (siehe Beispiel 1).

Bei Verwendung von Methanol als Lösungsmittel und Natriumhydroxid anstelle von Ammoniak werden nach dem Verfahren der US 5,151,543 vergleichbare Ausbeuten (63%) und Umsätze erzielt wie beim Einsatz von Ammoniak (Beispiel 3). Beim Einsatz eines Alkohols und eines Hydroxids als Base sind neben der unbefriedigenden Ausbeute die großen Mengen an Lösungsmittel nachteilig, da das Dinitril nur in einer Menge von etwa 10 Gew.-% eingesetzt werden kann, während bei der Verwendung von Ammoniak ungefähr die fünffache Menge (47 Gew.-%) eingesetzt werden kann. Zudem ist die Verwendung eines weiteren Hilfsstoffes (Alkohol) von Nachteil, da dieser nach der Reaktion vollständig entfernt werden muß, wenn man als Aminonitril 6-Aminocapronitril herstellt, das zu Caprolactam cyclisiert werden kann.

Nach dem Verfahren der WO 93/16034 kann man die Ausbeute an Aminocapronitril dadurch steigern, daß man Adiponitril in Gegenwart von Raney-Nickel, einer Base wie Natrium-, Kalium-, Lithium- oder Ammoniumhydroxid und einer Übergangsmetall-Komplexverbindung, mit beispielsweise Eisen, Cobalt, Chrom oder Wolfram als Übergangsmetallen, und eines Lösungsmittels hydriert. Nach diesem Verfahren werden bei Umsätzen im Bereich von 45 bis 60% quantitative Ausbeuten an Aminocapronitril beschrieben. Nachteilig an diesem Verfahren ist die Aufarbeitung der zumeist toxischen Übergangsmetall-Komplexverbindungen aus den erhaltenen Reaktionsgemischen.

Aufgabe der vorliegenden war daher die Bereitstellung eines verbesserten Verfahrens zur Herstellung von aliphatischen alpha,omega-Aminonitrilen durch partielle Hydrierung von Adipodinitril, das die zuvor genannten Nachteile nicht aufweist.

Demgemäß wurde ein Verfahren zur Herstellung von aliphatischen alpha,omega-Aminonitrilen durch partielle Hydrierung von aliphatischen alpha,omega-Dinitrilen bei erhöhter Temperatur und erhöhtem Druck in Gegenwart einer Base und eines Hydrierungs-Katalysators gefunden, indem man die Hydrierung in Gegenwart von Ammoniak und Lithiumhydroxid, oder Ammoniak und einer Verbindung, die während der Hydrierung Lithiumhydroxid liefert, durchführt.

Als Ausgangsstoffe im erfindungsgemäßen Verfahren werden aliphatische alpha,omega-Dinitrile der allgemeinen Formel I

NC-(CH₂)ₙ-CN I

in der n eine ganze Zahl von 1 bis 10, insbesondere 2, 3, 4, 5 und 6, bedeutet, eingesetzt. Besonders bevorzugte Verbindungen I sind Bernsteinsäuredinitril, Glutarsäuredinitril, Adipinsäuredinitril ("Adiponitril"), Pimelinsäuredinitril und Korksäuredinitril ("Suberonitril"), ganz besonders bevorzugt Adiponitril.

Nach dem erfindungsgemäßen Verfahren werden die vorstehend beschriebenen Dinitrile I in Gegenwart von Ammoniak und Lithiumhydroxid, oder einer unter den Reaktionsbedingungen Lithiumhydroxid-liefernden Verbindung, unter Verwendung eines Hydrierungs-Katalysators partiell zu alpha,omega-Aminonitrilen der allgemeinen Formel II

NC-(CH₂)ₙ-CH₂-NH₂ II

hydriert, wobei n die vorstehend genannte Bedeutung hat. Besonders bevorzugte Aminonitrile II sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril"), 7-Aminoheptansäurenitril und 8-Aminooctansaurenitril, ganz besonders bevorzugt 6-Aminocapronitril.

Die Umsetzung nimmt man bei Temperaturen im Bereich von 40 bis 120, vorzugsweise von 50 bis 100, besonders vorzugsweise von 60 bis 90°C vor; den Druck wählt man im allgemeinen im Bereich von 2 bis 12, vorzugsweise von 3 bis 10, besonders bevorzugt von 4 bis 8 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise beim Einsatz von Adiponitril im Bereich von 50 bis 275, vorzugsweise von 70 bis 200 min.

Bevorzugt wählt man die Druck- und Temperaturbereiche so, daß man die Umsetzung in flüssiger Phase vornehmen kann.

Ammoniak setzt man im allgemeinen in einer Menge ein, so daß das Gewichtsverhältnis von Ammoniak zu Dinitril im Bereich von 9:1 bis 0,1:1, bevorzugt von 2,3:1 bis 0,25:1, besonders bevorzugt von 1,5:1 bis 0,4:1, liegt.

Die Menge an Lithiumhydroxid wählt man in der Regel im Bereich von 0,1 bis 20, bevorzugt von 1 bis 10 Gew.-%, bezogen auf die Menge an eingesetztem Katalysator.

Als Lithiumverbindungen, die unter den Reaktionsbedingungen Lithiumhydroxid bilden, seien genannt: Lithiummetall, Alkyl- und Aryllithiumverbindungen wie n-Butyllithium und Phenyllithium. Die Menge an diesen Verbindungen wählt man im allgemeinen so, daß die zuvor genannte Menge an Lithiumhydroxid erhalten wird.

Als Katalysatoren setzt man bevorzugt Nickel-, Ruthenium-, Rhodium- und Cobalt-haltige Verbindungen ein, bevorzugt solche vom Raney-Typ, insbesondere Raney-Nickel und Raney-Cobalt. Man kann die Katalysatoren auch als Trägerkatalysatoren einsetzen, wobei als Träger beispielsweise Aluminiumoxid, Siliciumdioxid, Zinkoxid, Aktivkohle oder Titandioxid dienen können. (s. Appl. Het. Cat., 1987, S. 106-122; Catalysis, Vol. 4 (1981) S. 1-30). Besonders bevorzugt ist Raney-Nickel (beispielsweise von BASF AG, Degussa und Grace).

Die Nickel-, Ruthenium-, Rhodium- und Cobalt-Katalysatoren können mit Metallen der Gruppe VIB (Cr, Mo, W) und VIII (Fe, Ru, Os, Co (nur im Falle von Nickel), Rh, Ir, Pd, Pt) des Periodensystems modifiziert sein. Nach bisherigen Beobachtungen führt der Einsatz von insbesondere modifizierten Raney-Nickel-Katalysatoren, beispielsweise mit Chrom und/oder Eisen modifiziert, zu höheren Aminonitril-Selektivitäten. (Herstellung siehe DE-A 2 260 978; Bull. Soc. Chem. 13 (1946) S. 208).

Die Menge an Katalysator wählt man im allgemeinen so, daß die Cobalt-, Ruthenium-, Rhodium- oder Nickel-Menge im Bereich von 1 bis 50, bevorzugt von 5 bis 20 Gew.-%, bezogen auf die eingesetzte Menge an Dinitril, beträgt.

Die Katalysatoren können als Festbettkatalysatoren in Sumpf- oder Rieselfahrweise oder, bevorzugt, als Suspensionskatalysatoren eingesetzt werden.

Nach dem erfindungsgemäßen Verfahren erhält man alpha,omega-Aminonitrile in hoher Ausbeute und in guten Selektivitäten. Die alpha,omega-Aminonitrile sind wichtige Ausgangsverbindungen zur Herstellung von cyclischen Lactamen, insbesondere 6-Aminocapronitril für Caprolactam.

### Beispiele

### Beispiel 1 (Vergleichsbeispiel analog zu WO 92/21650, Beispiel 1)

Reaktor: 300 ml Autoklav
Ansatz: 60 g Adiponitril ("ADN"), 7 g Ra-Ni (Raney-Nickel) (BASF, H 1-50, wasserfeucht, ca. 6 g reines Ra-Ni).

ADN und Ra-Ni wurden unter Argon eingefüllt, anschließend wurde der Autoklav verschlossen und mit 100 ml flüssigem Ammoniak versetzt. Die Durchmischung erfolgte mit Magnetrührer.

Nach Erhitzen auf 80°C (Eigendruck: ca. 30 bar) wurde der Gesamtdruck durch Wasserstoff auf 70 bar erhöht. Der Druck von 70 bar wurde durch ständiges Nachpressen von Wasserstoff gehalten. Nach 30, 60, 90, 120, 180, 240, 300 und 360 min wurden Proben entnommen und gaschromatographisch analysiert. Die Versuchsergebnisse sind Tabelle 1 zu entnehmen.

**Tabelle 1**

| Zeit [min] | ACN¹⁾[%] | HMD²⁾ [%] | Umsatz [%] | ACN-selekt. [%] |
|---|---|---|---|---|
| 30 | 45,8 | 6,1 | 52,5 | 87,2 |
| 60 | 61,4 | 12.4 | 74,9 | 82,0 |
| 90 | 63,9 | 23,4 | 89,9 | 71,1 |
| 120 | 57,5 | 34,0 | 95,8 | 60,0 |
| 180 | 31,4 | 61,7 | 100,0 | 31,4 |
| 240 | 17,7 | 74,5 | 100,0 | 17,7 |
| 300 | 7,8 | 83,0 | 100,0 | 7,8 |
| 360 | 2,9 | 86,9 | 100,0 | 2,9 |

| | | | | |
|---|---|---|---|---|
| ¹⁾ ACN = 6-Aminocapronitril; | | | | |
| ²⁾ HMD = Hexamethylendiamin | | | | |

### Beispiel 2

Reaktor: 300 ml Autoklav
Ansatz: 60 g ADN, 7 g Ra-Ni (BASF, H 1-50, wasserfeucht, ca. 6 g Ra-Ni), 0,1 g LiOH.

ADN, LiOH und Ra-Ni wurden unter Argon eingefüllt, anschließend wurde der Autoklav verschlossen und 100 ml NH₃ aufgepreßt. Die Durchmischung erfolgte mit Magnetrührer.

Nach Erhitzen auf 80°C (Eigendruck: ca. 30 bar) wurde der Gesamtdruck durch Wasserstoff auf 70 bar erhöht. Der Druck von 70 bar wurde durch ständiges Nachpressen von Wasserstoff gehalten. Nach 30, 60, 90, 120, 180, 240, 300 und 360 min wurden Proben entnommen und gaschromatographisch analysiert. Die Versuchsergebnisse finden sich in Tabelle 2.

**Tabelle 2**

| Zeit [min] | ACN [%] | HMD [%] | Umsatz [%] | ACN-selekt. [%] |
|---|---|---|---|---|
| 30 | 14,05 | 0,2 | 14,8 | 98,0 |
| 60 | 40,2 | 1,7 | 42,1 | 95,5 |
| 90 | 54,6 | 3,8 | 58,5 | 93,3 |
| 120 | 65,8 | 7,4 | 73,7 | 89,3 |
| 180 | 73,8 | 18,8 | 93,0 | 79,4 |
| 240 | 62,2 | 36,6 | 99,1 | 62,8 |
| 300 | 43,5 | 56,0 | 100,0 | 43,5 |
| 360 | 26,4 | 73,0 | 100,0 | 26,4 |

Der Vergleich zwischen den Beispielen 1 und 2 demonstriert, daß durch den Zusatz von Lithiumhydroxid bei gleichem Umsatz höhere Aminocapronitril-Selektivitäten erreicht werden.

### Beispiel 3 (Vergleichsbeispiel mit NaOH anstelle von LiOH)

Beispiel 2 wurde mit 0,2 g NaOH anstelle von 0,1 g LiOH unter ansonsten gleichen Versuchsbedingungen wiederholt. Die Ergebnisse finden sich in Tabelle 3.

**Tabelle 3**

| Zeit [min] | ACN [%] | HMD [%] | Umsatz [%] | ACN-Selekt. [%] |
|---|---|---|---|---|
| 30 | 31,2 | 1,0 | 50,2 | 62.2 |
| 60 | 45,6 | 2,6 | 67,5 | 67,6 |
| 90 | 53,9 | 4,5 | 78,4 | 68,6 |
| 120 | 59,5 | 7,3 | 87,3 | 68,2 |
| 180 | 60,7 | 14,0 | 94,9 | 64,0 |
| 240 | 56,5 | 22,4 | 98,3 | 57,5 |

Der Vergleich zwischen den Beispielen 1, 2 und 3 zeigt, daß der Zusatz von NaOH keine Steigerung der Aminocapronitril-Selektivität bewirkt.

## Patentansprüche

1. Verfahren zur Herstellung von aliphatischen alpha,omegaAminonitrilen durch partielle Hydrierung von aliphatischen alpha,omega-Dinitrilen bei erhöhter Temperatur und erhöhtem Druck in Gegenwart einer Base und eines Hydrierungs-Katalysators, dadurch gekennzeichnet, daß man die Hydrierung in Gegenwart von Ammoniak und Lithiumhydroxid, oder Ammoniak und einer Verbindung, die während der Hydrierung Lithiumhydroxid liefert, durchführt.

2. Verfahren nach Anspruch 1, dadurch gekennzeichnet, daß man die Hydrierung bei einer Temperatur im Bereich von 40 bis 120°C vornimmt.

3. Verfahren nach den Ansprüchen 1 oder 2, dadurch gekennzeichnet, daß man die Hydrierung bei einem Druck im Bereich von 2 bis 12 MPa durchführt.

4. Verfahren nach den Ansprüchen 1 bis 3, dadurch gekennzeichnet, daß man als aliphatisches alpha,omega-Dinitril Adiponitril einsetzt unter Erhalt von 6-Aminocapronitril.

## Claims

1. A process for the preparation of aliphatic alpha,omega-aminonitriles by partial hydrogenation of aliphatic alpha,omegadinitriles at elevated temperatures and superatmospheric pressure in the presence of a base and of a hydrogenation catalyst, which comprises carrying out the hydrogenation in the presence of ammonia and lithium hydroxide or of ammonia and a compound which gives lithium hydroxide during the hydrogenation.

2. A process as claimed in claim 1, wherein the hydrogenation is carried out at from 40 to 120°C.

3. A process as claimed in claim 1 or 2, wherein the hydrogenation is carried out at from 2 to 12 MPa.

4. A process as claimed in any of claims 1 to 3, wherein the aliphatic alpha,omega-dinitrile used is adiponitrile, 6-aminocapronitrile being obtained.

## Revendications

1. Procédé de préparation d'aminonitriles-alpha,oméga aliphatiques par hydrogénation partielle de dinitriles-alpha,oméga aliphatiques à température élevée et pression élevée, en présence d'une base et d'un catalyseur d'hydrogénation, caractérisé en ce qu'on effectue l'hydrogénation en présence d'ammoniac et d'hydroxyde de lithium, ou d'ammoniac et d'un composé, qui fournit de l'hydroxyde de lithium pendant l'hydrogénation.

2. Procédé suivant la revendication 1, caractérisé en ce qu'on effectue l'hydrogénation à une température de l'ordre de 40 à 120°C.

3. Procédé suivant l'une des revendications 1 et 2, caractérisé en ce qu'on effectue l'hydrogénation à une pression de l'ordre de 2 à 12 MPa.

4. Procédé suivant l'une des revendications 1 à 3, caractérisé en ce que, comme dinitrile-alpha,oméga aliphatique, on met en oeuvre de l'adiponitrile, en obtenant du 6-aminocapronitrile.
